# EUROPEAN PATENT SPECIFICATION

(11) **EP 3 922 210 B1**
(45) Date of publication and mention of the grant of the patent: **02.10.2024**
(21) Application number: 20753192.2
(22) Date of filing: 10.02.2020
(51) Int. Cl.: A61C 13/00, A61C 13/34, A61C 9/00, A61C 19/05

(54) **DENTAL SYSTEM HAVING BASELINE ENABLING DIGITAL THREE-DIMENSIONAL TOOTH MODEL TO BE MERGED WITH ANATOMICAL LOCATION AND PLANE ANALYSIS OF HUMAN BODY**
ZAHNÄRZTLICHES SYSTEM MIT GRUNDLINIE ZUM ERMÖGLICHEN DES ZUSAMMENFÜHRENS EINES DIGITALEN DREIDIMENSIONALEN ZAHNMODELLS MIT ANATOMISCHER LAGE UND EBENENANALYSE DES MENSCHLICHEN KÖRPERS
SYSTÈME DENTAIRE AYANT UNE LIGNE DE BASE PERMETTANT DE FUSIONNER UN MODÈLE DE DENT NUMÉRIQUE TRIDIMENSIONNEL AVEC UN EMPLACEMENT ANATOMIQUE ET UNE ANALYSE DE PLAN DE CORPS HUMAIN

(30) Priority: 09.02.2019 KR 20190015250
(43) Date of publication of application: 15.12.2021
(73) Proprietor: Lee, Woo Hyoung, Yangju-si, Gyeonggi-do 11486 (KR)
(72) Inventor: Lee, Woo Hyoung, Yangju-si, Gyeonggi-do 11486 (KR)
(74) Representative: Frenkel, Matthias Alexander
(86) International application number: PCT/KR2020/001825
(87) International publication number: WO 2020/162730

(56) References cited:
- WO-A1-2019/027108
- KR-A- 20080 048 562
- KR-A- 20170 113 300
- KR-A- 20180 126 015
- KR-A- 20180 126 015
- KR-A- 20190 006 428
- KR-A- 20190 006 428
- KR-B1- 101 452 718
- US-A1- 2015 305 838
- US-A1- 2016 008 106

## Description

### BACKGROUND

### Field

The present disclosure relates to a dental system with a baseline so that a digital three-dimensional teeth model can be combined with an anatomical location and a plane analysis of the human body, and more particularly, to a dental system with a baseline so that a digital three-dimensional teeth model can be combined with an anatomical location and a plane analysis of the human body, in which a digital three-dimensional teeth model required for design of a digital dental prosthesis required to anodontia patients having no teeth or partial anodontia patients is modularized with a baseline so as to be placed at an anatomically ideal location, so that the digital three-dimensional teeth model is formed to rapidly and accurately engage with an anatomically ideal location, thereby chewing and pronouncing well.

### Description of the Related Art

Documents in the art are exemplified by KR 2019 0006428 A and KR 2018 0126015 A.

Generally, for humans, teeth are deeply related to extremely important functions such as eating, vocalization, facial expression in the mouth, and the loss of teeth is a deadly problem that damages these important functions.

A dental prosthesis is to rebuild a structure in the mouth which has been lost teeth.

At this time, as a method of producing artificial teeth, a method of using an oral scanner, a 3D scanner has been frequently used.

The three-dimensional teeth model is made through a three-dimensional printer and a three-dimensional processor through a process of scanning teeth and gums with an oral scanner, which is a kind of 3D scanner.

In other words, the patient's teeth and gums are created to virtual 3D teeth model data with an oral scanner or a teeth model scanner, the 3D data is output to an artificial teeth design program, and artificial teeth are designed based thereon. The artificial teeth designed by the artificial teeth design program are processed by a 3D processor to reproduce a shape of the artificial teeth. The artificial teeth produced as such need to be adjusted to be fitted to the teeth model well, and adjusted to be fitted to facing teeth so as to perform the functions in the mouth. In this process, a 3D artificial teeth design CAD program is required, and this process is called a digital prosthetic design.

The 3D dental prosthesis designed as such is designed to be placed at a unique anatomical location of the human body, and at this time, the individual teeth of the upper and lower jaws are essential to form the ideal engagement and reproduce the chewing and pronunciation well.

Accordingly, the process of digitizing the teeth and gums using the digital teeth design CAD program is a very important process to accurately reproduce the locations and forms of the tissues and teeth in the mouth required for producing artificial teeth.

However, in the related art, when the artificial teeth are fabricated with reference to a facial shape or shapes of upper and lower jaw bones which are photographed by a dental CT, x-ray, or the like to be output to an image, there is an inconvenience issue of work, such as adjusting each location one by one without being placed at an anatomical ideal location.

Accordingly, in the related art, there is a problem that it is difficult to align the artificial teeth at accurate locations without a baseline capable of locating the teeth at an anatomically ideal location in the process of designing the artificial teeth using a CAD program, and it takes a long time because the teeth are aligned one by one.

As a related prior art, there are Korean Patent Registration No. 10-1805003 (Title of Invention: Dental CAD system, method of driving the same, and recording medium, issued at November 29, 2017) and Korean Patent Registration No. 10-0583183 (Title of Invention: Method for providing data for straightening teeth, issued at May 18, 2006).

### SUMMARY

An object to be achieved by the present disclosure is to provide a dental system with a baseline so that a digital three-dimensional teeth model can be combined with an anatomical location and a plane analysis of the human body, in which a digital three-dimensional teeth model required for design of a digital dental prosthesis required to anodontia patients having no teeth or partial anodontia patients is modularized with a baseline so as to be placed at an anatomically ideal location, so that the digital three-dimensional teeth model is formed to rapidly and accurately engage with an anatomically ideal location, thereby chewing and pronouncing well.

Other objects of the present disclosure may be understood through the features of the present disclosure and may be more clearly known through exemplary embodiments of the present disclosure, and may be realized by means and combinations in the appended claims.

The invention is defined by the appended claims.

According to the present disclosure, since a digital three-dimensional teeth model required for design of a digital dental prosthesis required to anodontia patients having no teeth or partial anodontia patients is modularized with a baseline so as to be placed at an anatomically ideal location, the digital three-dimensional teeth model is formed to rapidly and accurately engage with an anatomically ideal location, thereby chewing and pronouncing well.

Other effects of the present disclosure may be understood through the features of the present disclosure and may be more clearly known through exemplary embodiments of the present disclosure, and may be realized by means and combinations in the appended claims.

### BRIEF DESCRIPTION OF THE DRAWINGS

The above and other aspects, features and other advantages of the present disclosure will be more clearly understood from the following detailed description taken in conjunction with the accompanying drawings, in which:

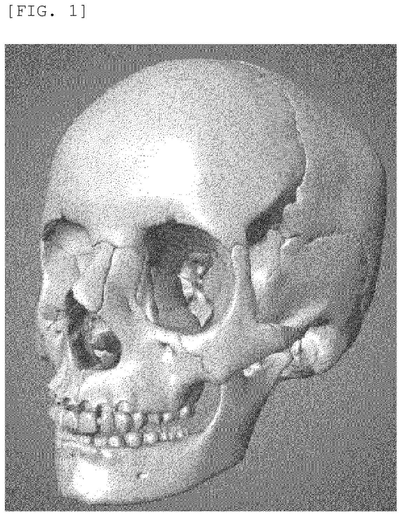

FIG. 1 is a perspective view of an exemplary embodiment of a digital 3D teeth model according to the related art;
FIG. 2 is a block diagram of an exemplary embodiment of a dental system with a baseline so that a digital three-dimensional teeth model can be combined with an anatomical location and a plane analysis of the human body according to the present disclosure;
FIG. 3 is a perspective view illustrating an acquisition unit according to FIG. 2;
FIG. 4 is a diagram illustrating a state in which an analysis unit according to FIG. 2 is disposed at an arch center which is a maxillary landmark;
FIG. 5 is a diagram illustrating a state in which the acquisition unit according to FIG. 2 is held between teeth of a face, a CT, and a plaster model of an anodontia patient;
FIG. 6 is a diagram illustrating a state in which the acquisition unit and the analysis unit according to FIG. 2 are combined with an anatomically ideal location of an anodontia patient;
FIG. 7 is a diagram illustrating CT data in the anodontia patient according to FIG. 2;
FIG. 8 is a diagram illustrating an acquisition unit having various heights according to FIG. 2;
FIG. 9 is a diagram illustrating a state in which various maxillary teeth according to FIG. 2 are disposed at the gum;
FIG. 10 is a diagram illustrating a state in which various mandibular teeth according to FIG. 2 are disposed at the gum;
FIG. 11 is a diagram illustrating a state in which maxillary/mandibular teeth and the gums according to FIG. 2 are controlled by a baseline of the analysis unit; and
FIG. 12 is a diagram illustrating a state in which the mandibular teeth and the gum according to FIG. 2 are separated from each other.

### DETAILED DESCRIPTION OF THE EMBODIMENT

The detailed description of the present disclosure to be described below refers to the accompanying drawings, which illustrate specific embodiments, as an example, in which the present disclosure may be implemented. These embodiments will be described in detail sufficient to enable those skilled in the art to implement the present disclosure. It should be understood that various embodiments of the present disclosure are different from each other, but need not be mutually exclusive. In the drawings, like reference numerals refer to the same or similar functions over several aspects.

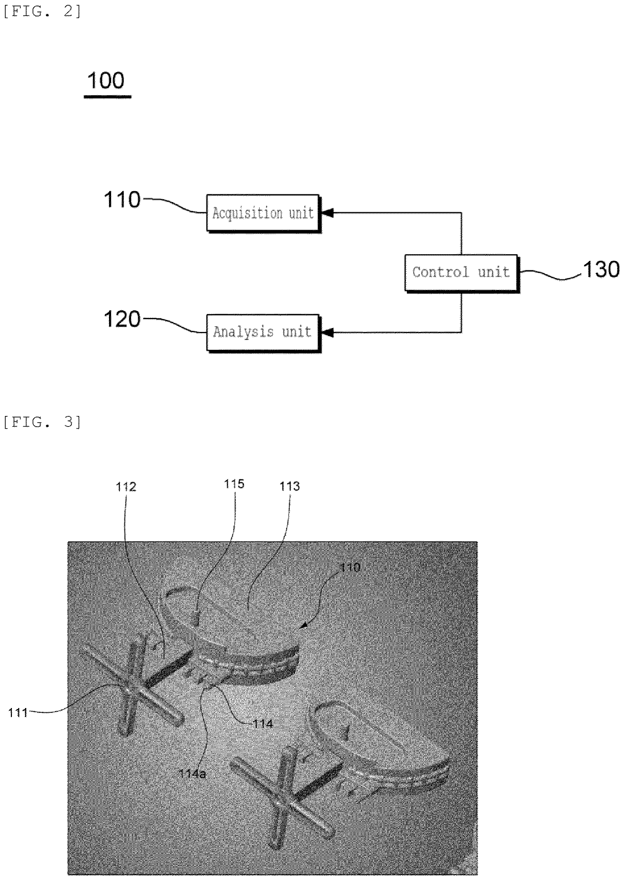

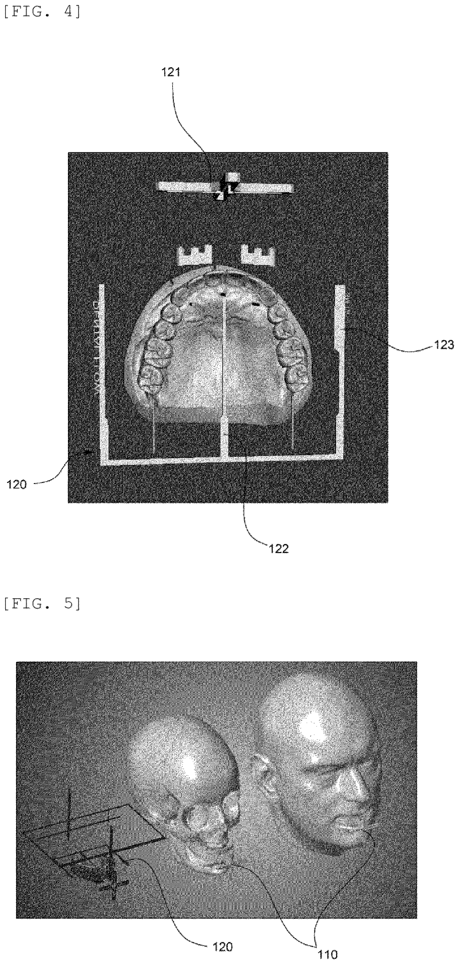

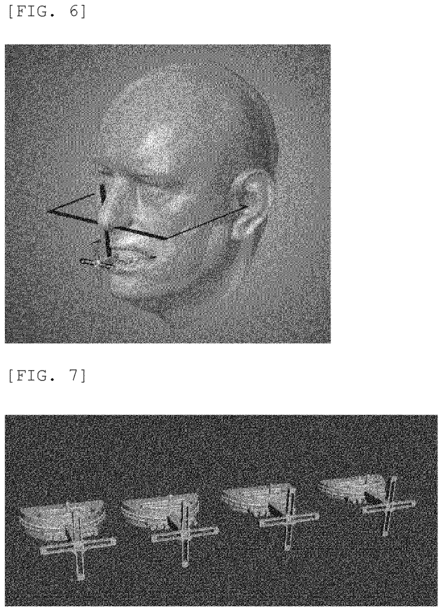

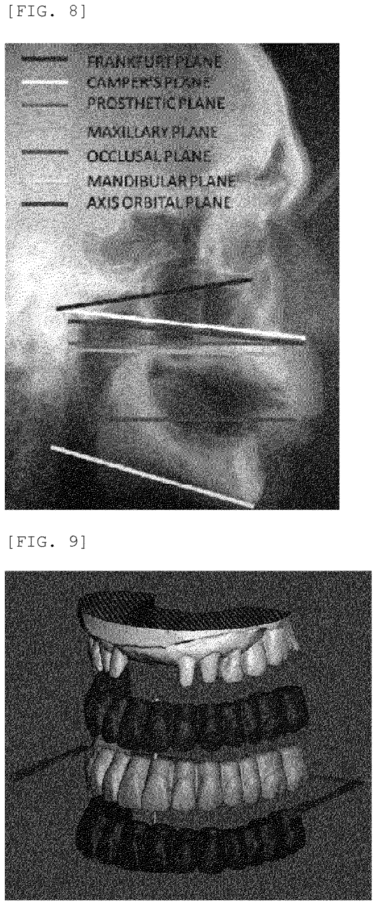

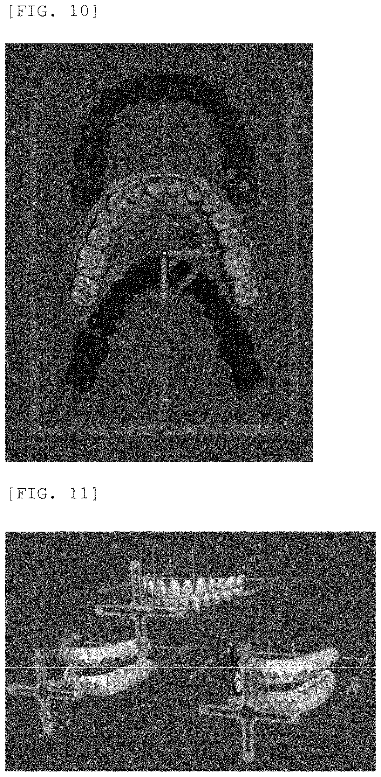

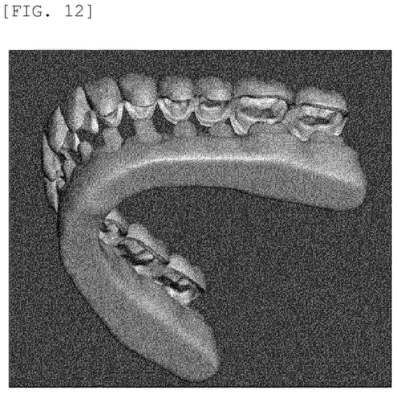

FIG. 2 is a block diagram of an exemplary embodiment of a dental system with a baseline so that a digital three-dimensional teeth model can be combined with an anatomical location and a plane analysis of the human body according to the present disclosure, FIG. 3 is a perspective view illustrating an acquisition unit according to FIG. 2, FIG. 4 is a diagram illustrating a state in which an analysis unit according to FIG. 2 is disposed at an arch center which is a maxillary landmark, FIG. 5 is a diagram illustrating a state in which the acquisition unit according to FIG. 2 is held between teeth of a face, a CT, and a plaster model of an anodontia patient, FIG. 6 is a diagram illustrating a state in which the acquisition unit and the analysis unit according to FIG. 2 are combined with an anatomically ideal location of an anodontia patient, FIG. 7 is a diagram illustrating CT data in the anodontia patient according to FIG. 2, FIG. 8 is a diagram illustrating an acquisition unit having various heights according to FIG. 2, FIG. 9 is a diagram illustrating a state in which various maxillary teeth according to FIG. 2 are disposed at the gum, FIG. 10 is a diagram illustrating a state in which various mandibular teeth according to FIG. 2 are disposed at the gum, FIG. 11 is a diagram illustrating a state in which maxillary/mandibular teeth and the gums according to FIG. 2 are controlled by a baseline of the analysis unit, and FIG. 12 is a diagram illustrating a state in which the mandibular teeth and the gum according to FIG. 2 are separated from each other.

A dental system 100 with a baseline so that a digital three-dimensional teeth model can be combined with an anatomical location and a plane analysis of the human body, as illustrated in FIGS. 2 to 12, includes an acquisition unit 110, an analysis unit 120, and a control unit 130.

The acquisition unit 110 is formed to be held by an anodontia patient or a partial anodontia patient in a mouth to acquire a tooth height or tooth distance of the anodontia patient or the partial anodontia patient.

The acquisition unit 110 acquires data acquired by a facial scanner, three-dimensional data acquired by a dental CT, and digital three-dimensional data acquired by scanning teeth of a plaster model, respectively.

The acquisition unit 110 is combined with an anatomically ideal location by the control unit 130 when moving to the anatomically ideal location together with the analysis unit 120 to be described below.

The acquisition unit 110 is configured by a base surface 111, a connection surface 112, a holding surface 113, and a scan protrusion surface 114.

The base surface 111 becomes a reference by crossing vertical and horizontal lines or surfaces.

In detail, the base surface 111 is formed in a vertical and horizontal shape, that is, a cross shape and provided at a constant interval with the holding surface 113 to be held by the anodontia patient in the mouth.

The base surface 111 always becomes a reference in an anatomically ideal location so that the acquisition unit 110 including the connection surface 112, the holding surface 113 and the scan protrusion surface 114 may be rapidly placed at an accurate location even if the acquisition unit 110 is moved to another location.

In the related art, there is a disadvantage that it takes a lot of time and the work is inconvenient when the acquisition unit 110 is placed at the anatomically ideal location without a separate reference when acquiring the tooth height or the tooth distance of the anodontia patient or the partial anodontia patient.

The acquisition unit 110 is combined with the analysis unit 120 to be described below through the base surface 111 of the acquisition unit 110 and a base surface 121 of the analysis unit 120 so as not to be misaligned with each other.

In the present disclosure, it is exemplified that the base surface 111 is formed in a cross shape to be a reference so that the acquisition unit 110 including the base surface 111 is rapidly placed at the accurate location, but the base surface 111 may be modified to other shapes as long as being a reference so that the acquisition unit 110 including the base surface 111 is rapidly placed at the accurate location.

The connection surface 112 is connected with a cross center of the base surface 111 in a bar shape.

In detail, the connection surface 112 is formed in a quadrangular bar shape so that one end is connected with a cross center of the base surface 111.

The connection surface 112 is formed in the quadrangular bar shape, but may be modified in other shapes such as a cylindrical shape so long as the connection surface 112 may maintain a distance between the base surface 111 and the holding surface 113.

The connection surface 112 maintains the distance between the base surface 111 and the holding surface 113 and is formed to be longitudinally adjusted in multi stages and the like according to the anodontia patient or the partial anodontia patient to adjust the distance between the base surface 111 and the holding surface 113.

The holding surface 113 has a hemispherical shape and is connected to one end corresponding to the one end of the connection surface 112 connected with the base surface 111 so as to be held by a maxillary gum and a mandibular gum of the anodontia patient or the remaining maxillary teeth and the remaining mandibular teeth of the partial anodontia patient in the mouth.

The holding surface 113 has a hemispherical shape, in which a round portion of the hemispherical shape is located at a lip portion of the anodontia patient or the partial anodontia patient and a straight portion thereof is located inside the mouth of the patient.

Grooves are formed on upper and lower surfaces of the holding surface 113, respectively, and when the patient holds the holding surface 113, a filling material such as silicone harmless to the human body is introduced into the groove.

A plurality of scan protrusion surfaces 114 is formed to protrude from the holding surface 113.

Specifically, the plurality of scan protrusion surfaces 114 is formed to protrude from the hemispherical holding surface 113, that is, an outer circumference of the round portion or formed to protrude in a direction of the base surface 111.

Among the scan protrusion surfaces 114, one end of the scan protrusion surface formed to protrude in the direction of the base surface 111 is exposed out of the patient's mouth to be accurately scanned.

The scan protrusion surface 114 is formed in a cylindrical shape or a square pillar shape, and a plurality of hemispherical protrusions 114a is formed on an outer circumference of the cylindrical shape or the square pillar shape.

In the related art, when scanned using a scanner, that is, an oral scanner, the scan protrusion surface cannot be accurately scanned due to a planar shape.

Accordingly, in the present disclosure, the hemispherical protrusion 114a is formed on the outer circumference of the scan protrusion surface 114 so as to be accurately scanned.

In the present disclosure, it is exemplified that the scan protrusion surfaces 114 are arranged in different shapes from each other such as the cylindrical shape and the square pillar shape, but in some cases, the scan protrusion surfaces 114 may be arranged in only the cylindrical shape or only the square pillar shape, that is, only the same shape.

A vertical protrusion surface 115 is included in the acquisition unit 110 and formed vertically in front of the upper surface of the holding surface 113 to be held by the maxillary gum of the anodontia patient so as to be fitted to a facial center line of the anodontia patient.

The vertical protrusion surface 115 is included in the acquisition unit 110 and formed vertically in front of the upper surface of the holding surface 113 to be held by the maxillary gum of the anodontia patient or the remaining maxillary teeth of the partial anodontia patient so as to be fitted to a facial center line of the anodontia patient.

The vertical protrusion surface 115 is formed on the upper surface of the holding surface 113 in a cylindrical shape to be in contact with a portion where the maxillary gum is first contacted.

The vertical protrusion surface 115 is formed on the upper surface of the holding surface 113 to fit a center line of the patient's face, and in some cases, may be formed even on a lower surface of the holding surface 113.

When the acquisition unit 110 is held by the anodontia patient or the partial anodontia patient, a filling material such as silicone harmless to the human body is filled in an empty space between the acquisition unit 110 and the gums of the anodontia patient or the remaining teeth of the partial anodontia patient.

Since the filling material is filled in the empty space between the acquisition unit 110 and the gums of the anodontia patient or the remaining teeth of the partial anodontia patient, the acquisition unit 110 is not moved to acquire accurately the tooth height, the tooth distance, or the like of the patient.

The acquisition unit 110 is fabricated to have a different height by measuring an average height due to a different unique height for each patient.

The analysis unit 120 is disposed at a maxillary landmark or a mandibular landmark of the anodontia patient or the partial anodontia patient to analyze an occlusal plane of the anodontia patient or the partial anodontia patient.

The analysis unit 120 consists of a base surface 121, a center surface 122, and an occlusal surface 123.

The base surface 121 becomes a reference by crossing vertical and horizontal lines or surfaces.

In detail, the base surface 121 is formed in a vertical and horizontal shape, that is, a cross shape and always becomes a reference in an anatomically ideal location so that the analysis unit 120 including the center surface 122 and the occlusal surface 123 may be rapidly placed at an accurate location even if the analysis unit 120 is moved to another location.

In the present disclosure, it is exemplified that the base surface 121 is formed in a cross shape to be a reference so that the analysis unit 120 including the base surface 121 is rapidly placed at the accurate location, but the base surface 121 may be modified to other shapes as long as being a reference so that the analysis unit 120 including the base surface 121 is rapidly placed at the accurate location.

The base surface 121 includes a form capable of analyzing a landmark in the mouth of the patient, and a form capable of analyzing an occlusal plane and a horizontal line connecting a vertical center line of the face and pupils of eyes out of the mouth of the facial skin or skull, and the mandible of the human body.

The center surface 122 is connected with the base surface 121 so that one surface is disposed at an arch center which is a landmark of the anodontia patient or the partial anodontia patient.

The center surface 122 is elongated in a longitudinal direction and one end of the center surface 122 is disposed at a maxillary or mandibular arch center (middle line) which is a landmark of the anodontia patient or the partial anodontia patient.

The occlusal surface 123 is connected with the center surface 122, in which the occlusal surfaces 123 are disposed at both sides based on the center surface 122 to be connected to each other, and the occlusal surface 123 is disposed on an occlusal plane of the anodontia patient or the partial anodontia patient.

The occlusal surface 123 is elongated in a longitudinal direction and one end and the other end thereof are formed to be curved orthogonally in an upper direction.

The center of the occlusal surface 123 of which one end and the other end are formed to be curved orthogonally in the upper direction is connected with the center surface 122, and one end and the other end formed to be curved orthogonally in the upper direction are connected with the center surface 122 in parallel with each other.

Referring to FIG. 7, when viewed from the side surface of the anodontia patient, the occlusal surface 123 refers to an anatomical base in which a connection line of maxillary and mandibular teeth coincides with or is 15° inclined with a connection line of the ear and the nose (CAMPER'S PLANE) or the ear and the eye bag (FRANKFURT PLANE).

That is, the occlusal plane of the anodontia patient is configured at the same angle and the same form as an anatomical component that can be easily analyzed.

The acquisition unit 110 and the analysis unit 120 are combined with each other around the base surface 111 of the acquisition unit 110 and the base surface 121 of the analysis unit 120.

When the acquisition unit 110 is first placed at an anatomically ideal location of the anodontia patient, the analysis unit 120 is integrally combined with the acquisition unit 110 through the base surface 121.

The digital three-dimensional teeth model of the anodontia patient or the partial anodontia patient is fabricated in advance and consists of maxillary teeth and mandibular teeth and consists of a maxillary teeth and gum unit and a mandibular teeth and gum unit to be controlled to each other through the base surface 121 of the analysis unit 120, and has a form of a female pattern and a male pattern so that a base portion of the maxillary teeth and the mandibular teeth and the upper surface portion of the gum may be coupled to each other.

The teeth model of the anodontia patient or the partial anodontia patient is classified according to a form and a size of the arch and a dental shape, and a plurality of arch forms and dental shapes are output when the teeth model is output as an image.

The analysis unit 120 is fabricated to have different occlusal planes by measuring an average occlusal plane by varying a unique occlusal plane for each anodontia patient.

The control unit 130 is connected to the acquisition unit 110 and the analysis unit 120, respectively to control the acquisition unit 110 and the analysis unit 120 to be modularized simultaneously or selectively.

The control unit 130 controls the modularization temporarily to adjust the movement due to an unexpected event in the process of moving the acquisition unit 110 and the analysis unit 120 to an anatomically ideal location.

## Claims

1. A dental system (100) for designing a digital dental prosthesis, with a baseline for locating teeth at an anatomically ideal location so that a digital three-dimensional teeth model can be combined with an anatomical location and a plane analysis of a human body, the dental system comprising:
an acquisition unit (110) which is formed to be held by an anodontia patient or a partial anodontia patient in a mouth to acquire a tooth height or tooth distance of the anodontia patient or the partial anodontia patient;
an analysis unit (120) which is disposed at a maxillary landmark or a mandibular landmark of the anodontia patient or the partial anodontia patient to analyze an occlusal plane of the anodontia patient or the partial anodontia patient; and
a control unit (130) which is connected to the acquisition unit (110) and the analysis unit (120), respectively, to control the acquisition unit (110) and the analysis unit (120) to be modularized simultaneously or selectively,
wherein the acquisition unit (110) includes:
a base surface (111) which becomes a reference by crossing vertical and horizontal lines or surfaces,
a connection surface (112) which is connected with a cross center of the base surface (111) in a bar shape,
a holding surface (113) which has a hemispherical shape and is connected to one end corresponding to the one end of the connection surface (112) connected with the base surface (111) so as to be held by a maxillary gum and a mandibular gum of the anodontia patient or remaining maxillary teeth and remaining mandibular teeth of the partial anodontia patient in the mouth, and
a plurality of scan protrusion surfaces (114) which is formed to protrude from an outer circumference of the holding surface (113),
wherein the analysis unit (120) includes:
a base surface (121) which becomes a reference by crossing vertical and horizontal lines or surfaces,
a center surface (122) which is associated with the base surface (121) so that one surface is disposed at an arch center which is a landmark of the anodontia patient or the partial anodontia patient, and
occlusal surfaces (123) which is connected with the center surface (122), in which the occlusal surfaces (123) are disposed at both sides based on the center surface (122) to be connected to each other, and is disposed on an occlusal plane of the anodontia patient or the partial anodontia patient, and
wherein the acquisition unit (110) and the analysis unit (120) are adapted to be combined with each other around the base surface (111) of the acquisition unit (110) and the base surface (121) of the analysis unit (120).

2. The dental system (100) of claim 1, wherein the acquisition unit (110) and the analysis unit (120) are combined with an anatomically ideal location by the control unit (130) when moving to the anatomically ideal position.

3. The dental system (100) of claim 1, wherein the plurality of scan protrusion surfaces (114) is formed to protrude from the outer circumference of the holding surface (113) or formed to protrude in a direction of the base surface (111).

4. The dental system (100) of claim 1, wherein the acquisition unit (110) further includes a vertical protrusion surface (115) which is formed vertically in front of an upper surface of the holding surface (113) to be held by the maxillary gum of the anodontia patient or the remaining maxillary teeth of the partial anodontia patient so as to be fitted to a facial center line of the anodontia patient or the partial anodontia patient.

5. The dental system (100) of claim 1, wherein the scan protrusion surface (114) is formed in a cylindrical shape or a square pillar shape, and a plurality of hemispherical protrusions (114a) are formed on an outer circumference of the cylindrical shape or the square pillar shape.

6. The dental system (100) of claim 1, wherein grooves are formed on upper and lower surfaces of the holding surface (113), respectively, and when the patient holds the holding surface (113), a filling material such as silicone harmless to the human body is introduced into the groove.

7. The dental system (100) of claim 1, wherein the base surface (121) includes a form capable of analyzing the landmark in the mouth of the patient, and a form capable of analyzing an occlusal plane and a horizontal line connecting a vertical center line of a face and pupils of eyes out of the mouth of the facial skin or skull, and the mandible of the human body.

8. The dental system (100) of claim 1, wherein when the acquisition unit (110) is held by the anodontia patient or the partial anodontia patient, a filling material such as silicone harmless to the human body is filled in an empty space between the acquisition unit (110) and gums of the anodontia patient or remaining teeth of the partial anodontia patient.

9. The dental system (100) of claim 1, wherein the acquisition unit (110) is fabricated to have a different height by measuring an average height due to a different unique height for each patient.

10. The dental system (100) of claim 1, wherein the digital three-dimensional teeth model of the anodontia patient or the partial anodontia patient is fabricated in advance and consists of maxillary teeth and mandibular teeth and consists of a maxillary teeth and gum unit and a mandibular teeth and gum unit to be controlled to each other through a base surface (121) of the analysis unit (120), and has a form of a female pattern and a male pattern so that a base portion of the maxillary teeth and the mandibular teeth and an upper surface portion of the gum may be coupled to each other.

11. The dental system (100) of claim 1, wherein the teeth model of the anodontia patient or the partial anodontia patient is classified according to a form and a size of an arch and a dental shape, and a plurality of arch forms and the dental shapes are output when the teeth model is output as an image.

## Patentansprüche

1. Zahnmedizinisches System (100) zum Entwerfen einer digitalen Zahnprothese, mit einer Basislinie zum Lokalisieren von Zähnen an einer anatomisch idealen Stelle, so dass ein digitales dreidimensionales Zahnmodell mit einer anatomischen Stelle und einer Ebenenanalyse eines menschlichen Körpers kombiniert werden kann, wobei das zahnmedizinische System umfasst:
eine Erfassungseinheit (110), die ausgebildet ist, von einem Anodontie-Patienten oder einem Teil-Anodontie-Patienten in einem Mund gehalten zu werden, um eine Zahnhöhe oder einen Zahnabstand des Anodontie-Patienten oder des Teil-Anodontie-Patienten zu erfassen;
eine Analyseeinheit (120), die an einem Oberkieferorientierungspunkt oder einem Unterkieferorientierungspunkt des Anodontie-Patienten oder des Teil-Anodontie-Patienten angeordnet ist, um eine Okklusionsebene des Anodontie-Patienten oder des Teil-Anodontie-Patienten zu analysieren; und
eine Steuereinheit (130), die mit der Erfassungseinheit (110) und der Analyseeinheit (120) jeweils verbunden ist, um die Erfassungseinheit (110) und die Analyseeinheit (120) zu steuern, damit sie gleichzeitig oder selektiv modularisiert werden,
wobei die Erfassungseinheit (110) umfasst:
eine Grundfläche (111), die durch Kreuzen von vertikalen und horizontalen Linien oder Flächen zu einer Referenz wird,
eine Verbindungsfläche (112), die mit einer Quermitte der Grundfläche (111) stabförmig verbunden ist,
eine Haltefläche (113), die eine halbkugelförmige Form hat und mit einem Ende verbunden ist, das dem einen Ende der Verbindungsfläche (112) entspricht, die mit der Grundfläche (111) verbunden ist, so dass sie von einem Oberkieferzahnfleisch und einem Unterkieferzahnfleisch des Anodontie-Patienten oder den verbleibenden Oberkieferzähnen und den verbleibenden Unterkieferzähnen des Teilanodontie-Patienten im Mund gehalten wird, und
eine Vielzahl von Abtastvorsprungsflächen (114), die so ausgebildet sind, dass sie von einem Außenumfang der Haltefläche (113) vorstehen,
wobei die Analyseeinheit (120) umfasst:
eine Grundfläche (121), die durch Kreuzen von vertikalen und horizontalen Linien oder Flächen zu einer Referenz wird,
eine Mittelfläche (122), die mit der Grundfläche (121) verbunden ist, so dass eine Fläche in einer Mitte eines Bogens angeordnet ist, der ein Orientierungspunkt des Anodontie-Patienten oder des Teil-Anodontie-Patienten ist, und
Okklusionsflächen (123), die mit der Mittelfläche (122) verbunden sind, wobei die Okklusionsflächen (123) auf beiden Seiten basierend auf der Mittelfläche (122) angeordnet sind, um miteinander verbunden zu werden, und auf einer Okklusionsebene des Anodontie-Patienten oder des Teil-Anodontie-Patienten angeordnet sind, und
wobei die Erfassungseinheit (110) und die Analyseeinheit (120) so angepasst sind, dass sie um die Grundfläche (111) der Erfassungseinheit (110) und die Grundfläche (121) der Analyseeinheit (120) herum miteinander kombiniert werden können.

2. Zahnmedizinisches System (100) nach Anspruch 1, wobei die Erfassungseinheit (110) und die Analyseeinheit (120) durch die Steuereinheit (130) mit einer anatomisch idealen Position kombiniert werden, wenn sie sich in die anatomisch ideale Position bewegen.

3. Zahnmedizinisches System (100) nach Anspruch 1, wobei die Vielzahl von Abtastvorsprungsflächen (114) so ausgebildet sind, dass sie von dem Außenumfang der Haltefläche (113) vorstehen oder so ausgebildet sind, dass sie in Richtung der Grundfläche (111) vorstehen.

4. Zahnmedizinisches System (100) nach Anspruch 1, wobei die Erfassungseinheit (110) ferner eine vertikale Vorsprungsfläche (115) aufweist, die vertikal vor einer oberen Fläche der Haltefläche (113) ausgebildet ist, um von dem Oberkieferzahnfleisch des Anodontie-Patienten oder den verbleibenden Oberkieferzähnen des Teilanodontie-Patienten gehalten zu werden, um an eine Gesichtsmittellinie des Anodontie-Patienten oder des Teilanodontie-Patienten angepasst zu werden.

5. Zahnmedizinisches System (100) nach Anspruch 1, wobei die Abtastvorsprungsfläche (114) in einer zylindrischen Form oder einer quadratischen Säulenform ausgebildet ist und eine Vielzahl von halbkugelförmigen Vorsprüngen (114a) an einem Außenumfang der zylindrischen Form oder der quadratischen Säulenform ausgebildet sind.

6. Zahnmedizinisches System (100) nach Anspruch 1, wobei auf der Ober- bzw. Unterseite der Haltefläche (113) Rillen ausgebildet sind und bei Halten der Haltefläche (113) durch den Patienten ein für den menschlichen Körper unschädliches Füllmaterial, wie z.B. Silikon, in die Rille eingeführt wird.

7. Zahnmedizinisches System (100) nach Anspruch 1, wobei die Grundfläche (121) eine Form umfasst, die in der Lage ist, den Orientierungspunkt in de Mund des Patienten zu analysieren, und eine Form, die in der Lage ist, eine Okklusionsebene und eine horizontale Linie zu analysieren, die eine vertikale Mittellinie eines Gesichts und Pupillen von Augen außerhalb des Mundes der Gesichtshaut oder des Schädels und des Unterkiefers des menschlichen Körpers verbindet.

8. Zahnmedizinisches System (100) nach Anspruch 1, wobei, wenn die Erfassungseinheit (110) von dem Anodontie-Patienten oder dem Teilanodontie-Patienten gehalten wird, ein für den menschlichen Körper unschädliches Füllmaterial wie Silikon in einen leeren Raum zwischen der Erfassungseinheit (110) und dem Zahnfleisch des Anodontie-Patienten oder den verbleibenden Zähnen des Teilanodontie-Patienten gefüllt wird.

9. Zahnmedizinisches System (100) nach Anspruch 1, wobei die Erfassungseinheit (110) so hergestellt ist, dass sie eine unterschiedliche Höhe aufweist, indem sie eine durchschnittliche Höhe aufgrund einer für jeden Patienten unterschiedlichen individuellen Höhe misst.

10. Zahnmedizinisches System (100) nach Anspruch 1, wobei das digitale dreidimensionale Zahnmodell des Anodontie-Patienten oder des Teilanodontie-Patienten im Voraus hergestellt wird und aus Oberkieferzähnen und
Unterkieferzähnen besteht und aus einer Oberkieferzahn- und Zahnfleischeinheit und einer Unterkieferzahn- und Zahnfleischeinheit besteht, die über eine Grundfläche (121) der Analyseeinheit (120) miteinander gesteuert werden, und eine Form eines weiblichen Musters und eines männlichen Musters hat, so dass ein Basisabschnitt der Oberkieferzähne und der Unterkieferzähne und ein oberer Oberflächenabschnitt des Zahnfleisches miteinander gekoppelt werden können.

11. Zahnmedizinisches System (100) nach Anspruch 1, wobei das Zahnmodell des Anodontie-Patienten oder des Teil-Anodontie-Patienten gemäß einer Form und einer Größe eines Bogens und einer Zahnform klassifiziert wird und eine Vielzahl von Bogenformen und Zahnformen ausgegeben werden, wenn das Zahnmodell als ein Bild ausgegeben wird.

## Revendications

1. Système dentaire (100) destiné à concevoir une prothèse dentaire numérique, avec une ligne de base pour localiser les dents dans un emplacement idéal en termes d'anatomie de sorte qu'un modèle de dents tridimensionnel puisse être combiné avec un emplacement anatomique et une analyse de plan d'un corps humain, le système dentaire comprenant:
une unité d'acquisition (110) qui est formée pour être tenue par un patient souffrant d'anodontie ou un patient souffrant d'anodontie partielle dans une bouche afin d'acquérir une hauteur de dent ou une distance de dent du patient en anodontie ou du patient en anodontie partielle;
une unité d'analyse (120) qui est disposée au niveau d'un repère maxillaire ou d'un repère mandibulaire du patient souffrant d'anodontie ou du patient souffrant d'anodontie partielle pour analyser un plan d'occlusion du patient souffrant d'anodontie ou du patient souffrant d'anodontie partielle ; et
une unité de commande (130) qui est connectée à l'unité d'acquisition (110) et à l'unité d'analyse (120), respectivement, pour commander l'unité d'acquisition (110) et l'unité d'analyse (120) afin qu'elles soient modulaires simultanément ou sélectivement,
dans lequel l'unité d'acquisition (110) comprend:
une surface de base (111) qui devient une référence en croisant des lignes ou des surfaces verticales et horizontales,
une surface de connexion (112) qui est reliée à un centre transversal de la surface de base (111) en forme de barre,
une surface de maintien (113) qui se présente sous forme hémisphérique et est reliée à une extrémité correspondant à l'extrémité de la surface de connexion (112) reliée à la surface de base (111) de manière à être maintenue par une gencive maxillaire et une gencive mandibulaire du patient souffrant d'anodontie ou par les dents maxillaires restantes et les dents mandibulaires restantes du patient souffrant d'anodontie partielle dans la bouche, et
une pluralité de surfaces saillantes de balayage (114) qui est formée pour faire saillie d'une circonférence extérieure de la surface de maintien (113),
dans lequel l'unité d'analyse (120) comprend:
une surface de base (121) qui devient une référence en croisant des lignes ou des surfaces verticales et horizontales,
une surface centrale (122) associée à la surface de base (121) de sorte qu'une surface est disposée au centre d'une arche qui est un point de repère du patient souffrant d'anodontie ou d'anodontie partielle, et
des surfaces occlusales (123) qui sont reliées à la surface centrale (122), dans lesquelles les surfaces occlusales (123) sont disposées de part et d'autre de la surface centrale (122) pour être reliées l'une à l'autre, et sont disposées sur un plan d'occlusion du patient souffrant d'anodontie ou du patient souffrant d'anodontie partielle, et
dans lequel l'unité d'acquisition (110) et l'unité d'analyse (120) sont conçues pour être combinées l'une à l'autre autour de la surface de base (111) de l'unité d'acquisition (110) et de la surface de base (121) de l'unité d'analyse (120).

2. Système dentaire (100) selon la revendication 1, dans lequel l'unité d'acquisition (110) et l'unité d'analyse (120) sont combinées à un emplacement anatomique idéal par l'unité de commande (130) lors du déplacement vers la position anatomique idéale.

3. Système dentaire (100) selon la revendication 1, dans lequel la pluralité de surfaces saillantes de balayage (114) est formée pour faire saillie de la circonférence extérieure de la surface de maintien (113) ou formée pour faire saillie dans une direction de la surface de base (111).

4. Système dentaire (100) selon la revendication 1, dans lequel l'unité d'acquisition (110) comprend en outre une surface saillante verticale (115) qui est formée verticalement devant une surface supérieure de la surface de maintien (113) qui doit être maintenue par la gencive maxillaire du patient souffrant d'anodontie ou par les dents maxillaires restantes du patient souffrant d'anodontie partielle, de manière à s'adapter à la ligne médiane du visage du patient souffrant d'anodontie ou du patient souffrant d'anodontie partielle.

5. Système dentaire (100) selon la revendication 1, dans lequel la surface de protubérance de balayage (114) a une forme cylindrique ou une forme de pilier carré, et plusieurs protubérances hémisphériques (114a) sont formées sur une circonférence extérieure de la forme cylindrique ou de la forme de pilier carré.

6. Système dentaire (100) selon la revendication 1, dans lequel des rainures sont formées sur les surfaces supérieure et inférieure de la surface de maintien (113), respectivement, et lorsque le patient tient la surface de maintien (113), un matériau de remplissage tel que du silicone inoffensif pour le corps humain est introduit dans la rainure.

7. Système dentaire (100) selon la revendication 1, dans lequel la surface de base (121) comprend une forme capable d'analyser le repère dans la bouche du patient, et une forme capable d'analyser un plan d'occlusion et une ligne horizontale reliant une ligne centrale verticale d'un visage et des pupilles des yeux en dehors de la bouche de la peau du visage ou du crâne, et de la mandibule du corps humain.

8. Système dentaire (100) selon la revendication 1, dans lequel lorsque l'unité d'acquisition (110) est tenue par le patient souffrant d'anodontie ou d'anodontie partielle, un matériau de remplissage tel que du silicone inoffensif pour le corps humain est placé dans un espace vide entre l'unité d'acquisition (110) et les gencives du patient souffrant d'anodontie ou les dents restantes du patient souffrant d'anodontie partielle.

9. Système dentaire (100) selon la revendication 1, dans lequel l'unité d'acquisition (110) est fabriquée pour avoir une hauteur différente en mesurant une hauteur moyenne en raison d'une hauteur unique différente pour chaque patient.

10. Système dentaire (100) selon la revendication 1, dans lequel le modèle numérique tridimensionnel des dents du patient souffrant d'anodontie ou du patient souffrant d'anodontie partielle est fabriqué à l'avance et se compose de dents maxillaires et de dents mandibulaires, d'une unité de dents maxillaires et de gencives et d'une unité de dents mandibulaires et de gencives à contrôler l'une par rapport à l'autre par l'intermédiaire d'une surface de base (121) de l'unité d'analyse (120), et présente un type féminin et un type masculin de sorte qu'une partie de la base des dents maxillaires et des dents mandibulaires et une partie de la surface supérieure de la gencive peuvent être accouplées l'une à l'autre.

11. Système dentaire (100) selon la revendication 1, dans lequel le modèle dentaire du patient souffrant d'anodontie ou du patient souffrant d'anodontie partielle est classé en fonction de la forme et de la taille d'une arcade et d'une forme dentaire, et une pluralité de formes d'arcade et de formes dentaires sont produites lorsque le modèle dentaire est produit sous forme d'image.
